# EUROPEAN PATENT SPECIFICATION

(11) **EP 0 691 812 B1**
(45) Date of publication and mention of the grant of the patent: **29.09.1999**
(21) Application number: 94912272.5
(22) Date of filing: 22.03.1994
(51) Int. Cl.: A01N 27/00, C12N 15/82

(54) **SOUTHERN CORN ROOTWORM CONTROL WITH LIMONENE**
BEKÄMPFUNG VON DIABROTICA UNDECIMPUNCTATA MITTELS LIMONEN
LUTTE CONTRE LES LARVES NUISIBLES AUX RACINES DU MAIS DU SUD DES ETATS-UNIS AU MOYEN DU LIMONENE

(30) Priority: 02.04.1993 US 42199
(43) Date of publication of application: 17.01.1996
(73) Proprietor: PIONEER HI-BRED INTERNATIONAL, INC., Des Moines, Iowa 50309 (US)
(72) Inventor: MEYER, Terry, E., Urbandale, IA 50322 (US)
(74) Representative: Irvine, Jonquil Claire
(86) International application number: US9403011
(87) International publication number: WO9422304

(56) References cited:
- DE-A- 3 734 657
- US-A- 4 379 168
- BIOLOGICAL ABSTRACTS, vol. 86, no. 4 1988, Philadelphia, PA, US; abstract no. 36599, L.L. KARR 'Insecticidal properties of dextro-limonene' & J PESTIC SCI, vol.13, no.2, 1988 pages 287 - 290

## Description

### Technical Field

This invention relates to providing maize and other plants which produce no or low levels of limonene with resistance to Southern Corn Rootworm and other insect pests by causing expression of limonene in the tissues of the plants in quantities sufficient to repel, kill, or inhibit those pests.

### Background of the Invention

Numerous insects are serious pests of common agricultural crops. One method of controlling insects has been to apply insecticidal organic, semiorganic or organometallic chemicals to crops. This method has numerous, art-recognized environmental and public health problems. A more recent method of control of insect pests has been the use of biological control organisms which are typically natural predators of the troublesome insects. These include other insects such as trachonid wasps, fungi such as milky-spore fungi, and bacteria such as Bacillus thuringiensis cv., commonly referred to as "Bt". However, it is difficult to apply biological control organisms to large areas, and even more difficult to cause those living organisms to remain in the treated area for an extended period. Still more recently, techniques in recombinant DNA have provided the opportunity to insert into plant cells cloned genes which express insecticidal toxins derived from biological control organisms such as Bt. This technology has given rise to additional concerns about eventual insect resistance to well-known, naturally occurring insect toxins, particularly in the face of heavy selection pressure, which may occur in some areas. Thus, a continuing need exists to identify naturally occurring insecticidal toxins which can be formed by plant cells directly by expression of a single structural gene which is not normally present in the plant.

Southern Corn Rootworm (Diabrotica undecimpunctata howardi Barber) is a particularly difficult pest to control or eradicate. It attacks the plant below the soil line, where insecticides are difficult or impossible to apply effectively. In addition, it is resistant to a number of otherwise effective chemical and biological control agents, including Bt toxins and some lectins.

Limonene, 1-methyl-4-(1-methylethenyl)cyclohexene; p-mentha-1,8-diene (Entry No. 5371, Merck Index 11th Ed.), occurs naturally in various ethereal oils, particularly oils of lemon, orange, caraway, dill and bergamot. It is a valuable industrial chemical. Some limonene is prepared by extraction from plants of the mint family, a large quantity is obtained from citrus oils, which are typically 80-90% limonene, and some is obtained from pine oil. It is also synthesized chemically and finds use as a solvent and cleaning agent (in the manufacture of synthetic pine oil), as an expectorant, as a wetting and dispersing agent, as a monomer in the manufacture of various polymeric resins, as a flavorant and a precursor in the synthesis of the flavorant carvone, and as a polymerization inhibitor in storage of the tetrafluoroethylene monomer used in the manufacture of polytetrafluoroethylene (PTFE).

### DISCLOSURE OF THE INVENTION

It has now been determined that limonene is especially effective in the control of Southern corn rootworm. Accordingly, this invention provides a method of killing, repelling or inhibiting larvae of Diabrotica undecimpunctata howardi, comprising administering enterally to the larvae an amount of limonene sufficient to be larvicidal or to inhibit the feeding of the larvae.

In this description of the invention the term "killing" has its usual meaning and is measured by counting larvae which die during a measurement period. "Inhibiting" is used to mean inhibition of larval growth, as measured by larval weight and weight gain of larvae which do not die during the measurement period, and "repelling" is used to mean non-preference for a plant treated in the manner of this invention as a food source even though growth may not be inhibited and death may not occur. Repellence is measured by comparing numbers of larvae infesting treated versus control plants.

In the practice of this invention limonene can be effectively applied to plants, harvested plant materials, or derived products consumed by the larvae by spray, microencapsulated beads, or other formulation common to the insecticidal arts. Care should be taken that the formulation is such that the limonene does not injure green tissues of the plant. Alternatively, the limonene can be incorporated into the tissues of a susceptible plant so that in the course of infesting the plant, the larvae consume larvicidal amounts of the limonene. One method of doing this is to incorporate limonene in a non-phytotoxic vehicle which is adapted for systemic administration to the susceptible plants. This method is commonly employed with insecticidal materials which are designed to attack chewing insects and is well within the purview of one of ordinary skill in the art of insecticide and larvicide formulation. Alternatively, a dietary bait containing limonene can be employed, with, optionally, an added pheromonal or other larval attractant material.

In view of the ability to transform crop plants to express various heterologous compounds, it would be desirable to transform maize plants to express limonene, so that by consuming the tissues of the plant the larvae would also consume toxic or inhibitory amounts of limonene. However, while most gene products are peptides, limonene is not a peptide or a peptide derivative and is not expressed from genes in the form of a peptide or peptide derivative, but is produced enzymatically as a secondary metabolite within the cells of some plants. We have now also found that the biosynthetic apparatus necessary for the production of limonene is present in many plant cells which do not produce limonene, including maize cells, with the exception of a single enzyme which can be produced through the expression of a single exogenous (heterologous) gene. That enzyme is limonene synthase, also known as limonene cyclase, which can directly synthesize limonene from geranyl pyprophosphate, which is found widely in both procaryotic and eucaryotic cells. Since genes which code for limonene synthase can be synthesized, either directly using a DNA sequence obtained by working backwards from the known amino acid sequence of limonene synthase and preferably using plant-preferred codons, or by cloning from natural sources of limonene, the resulting sequence can be inserted into an appropriate expression cassette, and introduced into cells of a susceptible plant species or a suitable endophytic bacterium, so that an especially preferred embodiment of this method involves inserting into the genome of the plant or bacterium a DNA sequence coding for limonene synthase, in proper reading frame relative to transcription initiator and promoter sequences active in the plant or bacterium. Transcription and translation of the DNA sequence under control of the regulatory sequences causes expression of the enzyme at levels which provide an insecticidal amount of limonene in the tissues of the plant which are normally infested by the larvae.

As an illustration, it can be noted that Colby et al., at the Keystone Symposium on Crop Improvement via Biotechnology: An International Perspective, Keystone, Colorado, USA, April 10-16, 1992, as reported in J. Cell Biochem. Suppl. 16, F, 230 (1992) have isolated and characterized cDNA encoding limonene cyclase from spearmint. To isolate and study the gene(s) (sic) encoding limonene synthase and to produce enough of the enzyme for structural studies, they used standard methods to extract RNA from young leaves of Mentha spicata and constructed a cDNA library in lambda ZAP XR (Stratagene) from poly (A)+ RNA. They designed three degenerate oligonucleotides based on internal amino acid sequences obtained from Edman degradation of purified limonene synthase and screened 250,000 clones to identify six positive clones that hybridized to all three oligonucleotides. The resulting clones could be used in the methods of this invention which involve plant transformation. However, Colby et al. indicate no appreciation of the value of the enzyme in conferring rootworm resistance to plants.

The plant which can be benefitted by this invention is preferably a plant susceptible to infestation and damage by the larvae of Diabrotica undecimpunctata howardi or whose harvested material is subject to attack by larvae of that insect. A prime example is corn (Zea mays). Transformation of maize cells, and the regeneration of transformed cells to produce whole, fertile, transformant (R0) offspring has also been reported by several centers and is now routine. However, this is not to be construed as limiting, inasmuch as this species has in the past been among the most difficult commercial crops to reliably transform and regenerate, and these insects (under other common names) also infest certain other crops. Thus the methods of this invention are readily applicable via conventional techniques to numerous plant species, if they are found to be susceptible to Diabrotica undecimpunctata howardi, including, without limitation, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum, and Datura.

Preferred plants that are to be transformed according to the methods of this invention are cereal crops, including maize, rye, barley, wheat, sorghum, oats, millet, rice, triticale, sunflower, alfalfa, rapeseed and soybean, fiber crops, such as cotton, fruit crops, such as melons, and vegetable crops, including onion, pepper, tomato, cucumber, squash, carrot, crucifer (cabbage, broccoli, cauliflower), eggplant, spinach, potato and lettuce.

The DNA sequence which when expressed imparts insecticidal activity is a structural gene which codes for limonene synthase. It has been found that limonene has sufficient insecticidal (larvicidal) activity to be operative when formed as the end product of a plant cell expression system. That is, while certain other compounds have some larvicidal activity at high concentrations in pure form, plant cell expression at such high concentrations is either not possible in a living plant cell system, or is not feasible if the commercially useful characteristics of the plant are to be preserved in terms of production of oils, starches, fibers, or other materials. Limonene, on the other hand, is not directly expressed as the gene product, and the peptide which is expressed in the methods of this invention is an enzyme which can catalyze the synthesis of large amounts of limonene for accumulation in the tissues of the transformed plant.

A tissue-specific promoter can be used in any instance where it may be desirable to localize production of limonene to an infested tissue or to a tissue which is efficient in production of the enzyme. Since Southern corn rootworm attack roots, an especially preferred tissue-specific promoter is a root-specific promoter.

In carrying out this invention, it will be appreciated that numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong constitutive promoter sequence at one end to cause the gene to be transcribed at a high frequency, and a poly-A recognition sequence at the other end for proper processing and transport of the messenger RNA. An example of such a preferred (empty) expression cassette into which the DNA sequence of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endonuclease recognition sites which may be cut in a predictable fashion by use of the appropriate enzyme. Such vectors are preferably constructed to include additional structural gene sequences imparting antibiotic or herbicide resistance, which then serve as selectable markers to identify and separate transformed cells. Preferred selection agents include kanamycin, chlorosulfuron, phosphonothricin, hygromycin and methotrexate, and preferred markers are genes conferring resistance to these compounds. A cell in which the foreign genetic material in a vector is functionally expressed has been "transformed" by the vector and is referred to as a "transformant".

A particularly preferred vector is a plasmid, by which is meant a circular double-stranded DNA molecule that is not a part of the chromosomes of the cell.

As mentioned above, genomic, synthetic and cDNA encoding the limonene synthase gene may be used in this invention. The vector of interest may also be constructed partially from a cDNA clone, partially from a synthetic sequence and partially from a genomic clone. When the limonene synthase gene sequence is in hand, genetic constructs are made which contain the necessary regulatory sequences to provide for efficient expression of the gene in the host cell. According to this invention, the genetic construct will contain (a) a first genetic sequence coding for the larvicidal compound of interest and (b) one or more regulatory sequences operably linked on either side of the limonene synthase structural gene. Typically, the regulatory sequences will be selected from the group comprising of promoters and terminators. The regulatory sequences may be from autologous or heterologous sources.

Promoters that may be used in the genetic sequence include nos, ocs, phaseolin, CaMV, FMV and other promoters isolated from the DNA of plants or other sources, both natural and synthetic.

An efficient plant promoter that may be used is an overproducing plant promoter. Overproducing plant promoters that may be used in this invention include the promoter of the small sub-unit (ss) of the ribulose-1,5-bisphosphate carboxylase from soybean (Berry-Lowe et al, J. Molecular and App. Gen., 1:483-498 (1982)), and the promoter of the cholorophyll a-b binding protein. However, these two promoters are known to be light-induced in eukaryotic plant cells (see, for example, Genetic Engineering of Plants, An Agricultural Perspective, Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)) and may be less desirable when root expression is desired. An especially preferred constitutive promoter is the 35S promoter from Cauliflower Mosaic Virus.

Root-specific promoters are especially preferred for the control of Southern corn rootworm while minimizing limonene production in the agronomically valuable parts of the plant. These promoters are also well known and can be selected from the many available from the literature or isolated de novo from various compatible species. For example, Hirel, B., Marsolier, M.C., Hoarau, A., Hoarau, J., Brangeon, J., Schafer, R., and Verma, D.P.S., Plant Molecular Biology, Oct 1992. v. 20 (2) p. 207-218, describe a root-specific glutamine synthetase gene from soybean. Keller, B. and Baumgartner, C., The Plant Cell, Oct 1991. v. 3 (10) p. 1051-1061, describe a root-specific control element in the GRP 1.8 gene of French bean. Sanger, M., Daubert, S., and Goodman, R.M., Plant Molecular Biology Mar 1990. v. 14 (3) p. 433-443, discuss the root-specific promoter of the Mannopine Synthase (MAS) gene of Agrobacterium tumefaciens. Miao, G.H., Hirel, B., Marsolier, M.C., Ridge, R.W., and Verma, D.P.S., The Plant Cell, Jan 1991. v. 3 (1) p. 11-22, describe a full-length cDNA clone encoding cytosolic glutamine synthetase (GS), which is expressed in roots and root nodules of soybean. Bogusz, D., Llewellyn, D.J., Craig, S., Dennis, E.S., Appleby, C.A., and Peacock, W.J., The Plant Cell, July 1990. v. 2 (7) p. 633-641, discusses two root-specific promoters isolated from hemoglobin genes from the nitrogen-fixing nonlegume Parasponia andersonii and the related non-nitrogen-fixing nonlegume Trema tomentosa. The promoters of these genes were linked to a beta-glucuronidase reporter gene and introduced into both the nonlegume Nicotiana tabacum and the legume Lotus corniculatus, and in both instances root-specific promoter activity was preserved. Leach, F. and Aoyagi, K., Plant Science (Limerick) 1991, 79 (1): 69-76, describe their analysis of the promoters of the highly expressed rolC and rolD root-inducing genes of Agrobacterium rhizogenes. They concluded that enhancer and tissue-specific DNA determinants are dissociated in those promoters. Teeri, T. H., Lehvaslaiho, H., Franck, M., Uotila, J., Heino, P., Palva, E. T., Montagu, M. van, and Herrera-Estrella, L., EMBO Journal, 1989, 8 (2): 343-350, used gene fusions to lacZ to show that the Agrobacterium T-DNA gene encoding octopine synthase is especially active in the epidermis of the root tip and that the TR2' gene was root specific in the intact plant and stimulated by wounding in leaf tissue, an especially desirable combination of characteristics for use with an insecticidal or larvicidal gene. The TR1' gene, fused to NPTII, (neomycin phosphotransferase II) showed similar characteristics.

The expression cassette comprising the structural gene for limonene synthase operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as E. coli, S. typhimurium, and S. marcescens. Eukaryotic hosts such as yeast or filamentous fungi may also be used in this invention.

The isolated cloning vector will then be introduced into the plant cell using any convenient technique, including electroporation (in protoplasts), retroviruses, microparticle bombardment, and microinjection, into cells from monocotyledonous or dicotyledonous plants, in cell or tissue culture, to provide transformed plant cells containing as foreign DNA at least one copy of the DNA sequence of the plant expression cassette. Preferably, the monocotyledonous species will be selected from maize, sorghum, wheat and rice, and the dicotyledonous species will be selected from soybean, sunflower, cotton, rapeseed (either edible or industrial), alfalfa, tobacco, and Solanaceae such as potato and tomato. Using known techniques, protoplasts can be regenerated and cell or tissue culture can be regenerated to form whole fertile plants which carry and express the desired gene for the selected protein. Accordingly, a highly preferred embodiment of the present invention is a transformed maize plant, the cells of which contain as foreign DNA at least one copy of the DNA sequence of an expression cassette which drives expression of limonene synthase.

This invention also provides methods of imparting resistance to Diabrotica undecimpunctata howardi to plants of a susceptible taxon, comprising the steps of:
a) culturing cells or tissues from at least one plant from the taxon,
b) introducing into the cells of the cell or tissue culture at least one copy of an expression cassette comprising a structural gene coding for limonene synthase, operably linked to plant regulatory sequences which cause the expression of the enzyme in the cells, and
c) regenerating insect-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the limonene synthase gene and associated regulatory sequences via crossing and backcrossing. Such intermediate methods will comprise the further steps of
a) sexually crossing the insect-resistant plant with a plant from the insect-susceptible taxon;
b) recovering reproductive material from the progeny of the cross; and
c) growing insect-resistant plants from the reproductive material. Where desirable or necessary, the agronomic characteristics of the susceptible taxon can be substantially preserved by expanding this method to include the further steps of repetitively:
a) backcrossing the insect-resistant progeny with insect-susceptible plants from the susceptible taxon; and
b) selecting for expression of insect resistance (or an associated marker gene) or limonene production among the progeny of the backcross, until the desired percentage of the characteristics of the susceptible taxon are present in the progeny along with the gene imparting insect resistance.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incorporated into Agrobacterium tumefaciens, which can then be used to transfer the vector into susceptible plant cells, primarily from dicotyledonous species. Thus, this invention provides a method for imparting insect resistance in Agrobacterium tumefaciens-susceptible dicotyledonous plants in which the expression cassette is introduced into the cells by infecting the cells with Agrobacterium tumefaciens, a plasmid of which has been modified to include a plant expression cassette which expresses limonene synthase in the manner of this invention.

Finally, insect pests of harvested material, including stored grain, can also be targets for the methods of this invention. In view of this, the invention also provides methods for killing larvae of Diabrotica undecimpunctata howardi in harvested materials and products obtained from harvested materials, comprising applying limonene to the grain or causing limonene synthase to be expressed in the grain.

The following description further exemplifies the compositions of this invention and the methods of making and using them. However, it will be understood that other methods, known by those of ordinary skill in the art to be equivalent, can also be employed.

### Examples 1-6

### Insect larvae inhibition and toxicity assays For Southern Corn Rootworm (SCR) and European Corn Borer (ECB)

Bioassay diets were prepared as described in Czapla and Lang in "Effect of Plant Lectins on the Larval Development of European Corn Borer (Lepidoptera: Pyralidae) and Southern Corn Rootworm (Coleoptera: Chrysomelidae)", J. Econ. Entomol., 83:2480-85 (1990), except that low melting temperature agarose replaced the regular agarose so that the diets could be chilled to 37°C prior to the addition of limonene (one assay used the regular agarose diet).

Results were as follows. In Examples 1-4, the test larvae were Southern Corn Rootworm. In Examples 5-6, the test larvae were European Corn Borer. The results of each experiment represent the average from 16-32 insects. All limonene concentrations (ppm) are by weight. The SCR data indicate that limonene is effective against the larvae, but when limonene was used in the same protocol against ECB, little or no effect was seen, indicating that limonene is ineffective against some common, relatively susceptible crop pests.

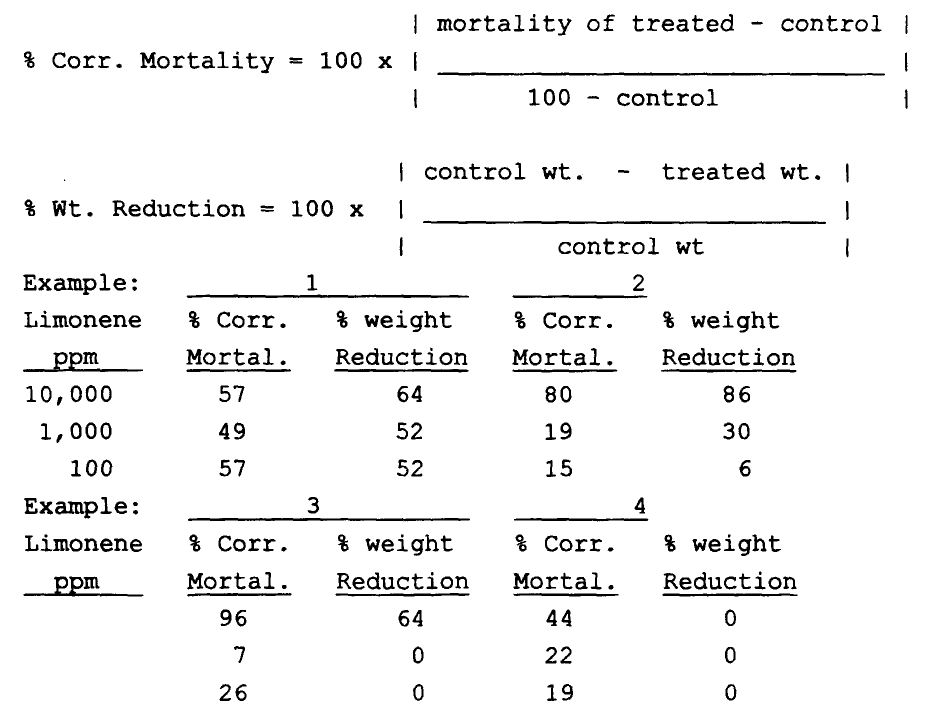

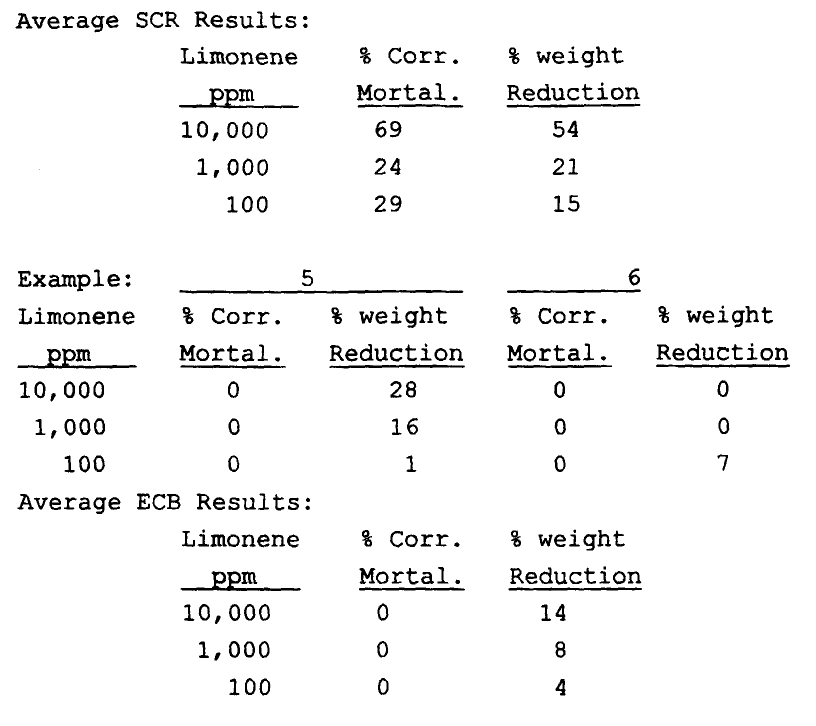

## Claims

1. A method of killing or inhibiting larvae of Diabrotica undecimpunctata howardi, comprising administering enterally to the larvae an amount of limonene sufficient to be larvicidal or to inhibit the feeding of the larvae.

2. A method according to Claim 1 wherein the compound is administered enterally by incorporating the compound in the diet of the larvae.

3. A method according to Claim 2 wherein the diet of the larvae comprises the tissues of a living plant.

4. A method according to Claim 3 for protecting a plant against infestation by larvae of Diabrotica undecimpunctata howardi comprising inserting into the genome of the plant at least one sequence coding for limonene synthase in proper reading frame relative to transcription initiator and promoter sequences active in the plant to cause expression of the enzyme at levels which provide a larvicidal amount of limonene in the tissues of the plant which are infested by the larvae.

5. A method according to Claim 4 wherein the plant is a monocotyledonous species selected from corn, wheat, rice, oats, rye, and sorghum.

6. A method according to Claim 4 wherein the plant is a dicotyledonous species selected from soybean, sunflower, rapeseed, alfalfa, cotton, melon, cucumber, lettuce, pepper and tomato.

7. A method according to claim 4 wherein the promoter sequence is a tissue-specific promoter.

8. A method according to Claim 7 wherein the tissue-specific promoter is a root-specific promoter.

9. A method of protecting corn plants (Zea mays) against damage caused by Southern corn rootworm (Diabrotica undecimpunctata howardi) comprising inserting into the genome of the corn plants a gene which codes for limonene synthase operatively linked to a root-specific promoter.

10. A method comprising:
a) culturing plant cells or tissues;
b) introducing into the cells or the cells of the tissue culture at least one copy of an expression cassette comprising a structural gene coding for limonene synthase, operably linked to plant regulatory sequences which cause the expression of the enzyme in the cells; and
c) regenerating insect-resistant whole plant from the cell or tissue culture, such that the enzyme is expressed at levels which provide an amount of limonene sufficient to be larvicidal to, or inhibit the feeding of, larvae of Diabrotica undecimpunctata howardi;
and optionally:
d) sexually or clonally reproducing the plants obtained in step (c) in such manner that at least one copy of the limonene synthase coding sequence provided by the expression cassette is present in the cells of progeny of the reproduction and such that the enzyme is expressed at levels which provide an amount of limonene sufficient to be larvicidal to, or inhibit the feeding of, larvae of Diabrotica undecimpunctata howardi.

11. A method according to claim 10 comprising:
a) culturing plant cells or tissues;
b) introducing into the cells of the cell or tissue culture at least one copy of an expression cassette comprising a structural gene coding for limonene synthase, operably linked to plant regulatory sequences which cause the expression of the enzyme in the cells; and
c) regenerating insect-resistant whole plants from the cell or tissue culture;
d) followed by sexually crossing the insect-resistant plant with a plant of an insect-susceptible taxon;
e) recovering reproductive material from the progeny of the cross; and
f) growing insect-resistant plants from the reproductive material;
and optionally:
g) backcrossing the insect-resistant progeny with plants of an insect-susceptible taxon; and
h) selecting for expression of insect resistance, or for an associated marker gene, or for limonene production among the progeny of the backcross;
until a desired percentage of the characteristics of the susceptible plants of the susceptible taxon are present along with the limonene synthase gene imparting insect resistance.

12. A method according to claim 10 or 11 wherein the plant is a monocotyledonous species selected from corn, wheat, rice, oats, rye, and sorghum.

13. A method according to claim 10 or 11 wherein the plant is a dicotyledonous species selected from soybean, sunflower, rapeseed, alfalfa, cotton, melon, cucumber, lettuce, pepper and tomato.

14. A method according to claim 10 or 11 wherein the promoter sequence is a tissue-specific promoter.

15. A method according to claim 14 wherein the tissue-specific promoter is a root-specific promoter.

## Patentansprüche

1. Verfahren zur Abtötung oder Hemmung von Larven von Diabrotica undecimpunctata howardi, umfassend die enterale Verabreichung einer Menge an Limonen, die ausreicht, larvizid zu sein oder die Futteraufnahme der Larven zu hemmen, an die Larven.

2. Verfahren nach Anspruch 1, wobei die Verbindung enteral durch Einarbeiten der Verbindung in das Futter der Larven verabreicht wird.

3. Verfahren nach Anspruch 2, wobei das Futter der Larven das Gewebe einer lebenden Pflanze umfaßt.

4. Verfahren nach Anspruch 3 zum Schutz einer Pflanze gegen Befall durch Larven von Diabrotica undecimpunctata howardi, umfassend die Insertion in das Genom der Pflanze mindestens einer Sequenz, die Limonensynthase codiert, im richtigen Leseraster, bezogen auf den Transkriptionsinitiator und die Promotorsequenzen, die in der Pflanze aktiv sind, um die Expression des Enzyms in Gehalten zu verursachen, die eine larvizide Menge an Limonen in den Geweben der Pflanze, die von den Larven befallen werden, ergeben.

5. Verfahren nach Anspruch 4, wobei die Pflanze eine monocotyle Art, ausgewählt aus Mais, Weizen, Reis, Hafer, Roggen und Sorghum, ist.

6. Verfahren nach Anspruch 4, wobei die Pflanze eine dicotyle Art, ausgewählt aus Sojabohnen, Sonnenblumen, Rapssamen, Alfalfa, Baumwolle, Melone, Gurke, Kopfsalat, Paprika und Tomate, ist.

7. Verfahren nach Anspruch 4, wobei die Promotorsequenz ein gewebsspezifischer Promotor ist.

8. Verfahren nach Anspruch 7, wobei der gewebsspezifische Promotor ein wurzelspezifischer Promotor ist.

9. Verfahren zum Schutz von Maispflanzen (Zea mays) gegen durch den Südlichen Maiswurzelwurm (Diabrotica undecimpunctata howardi) hervorgerufene Schäden, umfassend die Insertion eines Gens in das Genom der Maispflanzen, das Limonensynthase in funktioneller Verknüpfung an einen wurzelspezifischen Promotor codiert.

10. Verfahren, umfassend:
a) Züchtung von Pflanzenzellen oder Geweben,
b) Einschleusung in die Zellen oder die Zellen der Gewebskultur mindestens einer Kopie einer Expressionskassette, umfassend ein Strukturgen, das Limonensynthase codiert, in funktioneller Verknüpfung an Pflanzenregulatorsequenzen, die die Expression des Enzyms in den Zellen verursachen, und
c) Regenerieren insektenresistenter ganzer Pflanzen aus der Zell- oder Gewebskultur, so daß das Enzym in Gehalten exprimiert wird, die eine Menge an Limonen ergeben, die ausreicht, larvizid gegenüber den Larven von Diabrotica undecimpunctata howardi zu sein oder die Futteraufnahme zu hemmen,
und gegebenenfalls:
d) sexuelle oder clonale Reproduktion der in Stufe (c) erhaltenen Pflanzen so, daß mindestens eine Kopie der die Limonensynthase codierenden Sequenz, die durch die Expressionskassette bereitgestellt wird, in den Zellen der Nachkommen der Reproduktion vorhanden ist und so, daß das Enzym in Gehalten exprimiert wird, die eine Menge an Limonen ergeben, die ausreicht, larvizid gegenüber den Larven von Diabrotica undecimpunctata howardi zu sein oder die Futteraufnahme dieser Larven zu hemmen.

11. Verfahren nach Anspruch 10, umfassend:
a) Züchtung von Pflanzenzellen oder Geweben,
b) Einschleusung in die Zellen der Zell- oder Gewebskultur mindestens einer Kopie einer Expressionskassette, umfassend ein Strukturgen, das Limonensynthase codiert, in funktioneller Verknüpfung an Pflanzenregulatorsequenzen, die die Expression des Enzyms in den Zellen verursachen, und
c) Regenerieren insektenresistenter ganzer Pflanzen aus der Zell- oder Gewebskultur,
d) gefolgt von sexueller Kreuzung der insektenresistenten Pflanze mit einer Pflanze eines insektenempfindlichen Taxons,
e) Gewinnen des reproduktiven Materials aus den Nachkommen der Kreuzung und
f) Züchten insektenresistenter Pflanzen aus dem reproduktiven Material,
und gegebenenfalls
g) Rückkreuzen der insektenresistenten Nachkommen mit Pflanzen eines insektenempfänglichen Taxons und
h) Selektion nach Expression von Insektenresistenz oder eines assoziierten Markergens oder Limonenproduktion unter den Nachkommen der Rückkreuzung,
bis ein gewünschter Prozentsatz der Eigenschaften der empfindlichen Pflanzen des empfindlichen Taxons zusammen mit dem Limonensynthasegen, das Insektenresistenz verleiht, vorhanden ist.

12. Verfahren nach Anspruch 10 oder 11, wobei die Pflanze eine monocotyle Art, ausgewählt aus Mais, Weizen, Reis, Hafer, Roggen und Sorghum, ist.

13. Verfahren nach Anspruch 10 oder 11, wobei die Pflanze eine decotyle Art, ausgewählt aus Sojabohnen, Sonnenblumen, Rapssamen, Alfalfa, Baumwolle, Melone, Gurke, Kopfsalat, Paprika und Tomate, ist.

14. Verfahren nach Anspruch 10 oder 11, wobei die Promotorsequenz ein gewebsspezifischer Promotor ist.

15. Verfahren nach Anspruch 14, wobei der gewebsspezifische Promotor ein wurzelspezifischer Promotor ist.

## Revendications

1. Méthode destinée à tuer ou à inhiber des larves de Diabrotica undecimpunctata howardi, comprenant l'administration par voie entérique aux larves d'une quantité de limonène suffisante pour être larvicide ou inhiber la capacité des larves à s'alimenter.

2. Méthode selon la revendication 1 dans laquelle le composé est administré par voie entérique en incorporant le composé aux aliments des larves.

3. Méthode selon la revendication 2 dans laquelle les aliments des larves comprennent les tissus d'une plante vivante.

4. Méthode selon la revendication 3 destinée à protéger une plante contre l'infestation par des larves de Diabrotica undecimpunctata howardi comprenant l'insertion dans le génome de la plante d'au moins une séquence codant pour la limonène synthase dans le cadre de lecture correct par rapport à l'initiation de la transcription et des séquences promotrices actives dans la plante pour entraîner l'expression de l'enzyme à des niveaux qui produisent une quantité larvicide de limonène dans les tissus de la plante qui sont infestés par les larves.

5. Méthode selon la revendication 4 dans laquelle la plante est une espèce de Monocotylédones choisie parmi le maïs, le blé, le riz, l'avoine, le seigle et le sorgho.

6. Méthode selon la revendication 4 dans laquelle la plante est une espèce de Dicotylédones choisie parmi le soja, le tournesol, le colza, la luzerne, le coton, le melon, le concombre, la laitue, le poivron et la tomate.

7. Méthode selon la revendication 4 dans laquelle la séquence promotrice est un promoteur tissu-spécifique.

8. Méthode selon la revendication 7 dans laquelle la séquence promotrice tissu-spécifique est un promoteur spécifique de la racine.

9. Méthode de protection de plants de maïs (*Zea mays*) contre les dégâts causés par la chrysomèle des racines (Diabrotica undecimpunctata howardi), comprenant l'insertion dans le génome des plants de maïs d'un gène qui code pour la limonène synthase lié de manière fonctionnelle à un promoteur spécifique de la racine.

10. Méthode comprenant :
(a) la culture de cellules ou de tissus de plantes ;
(b) l'introduction dans les cellules ou dans les cellules de la culture de tissus d'au moins une copie d'une cassette d'expression comprenant un gène de structure codant pour la limonène synthase, lié de manière fonctionnelle à des séquences régulatrices de plante qui entraînent l'expression de l'enzyme dans les cellules ; et
(c) la régénération d'une plante entière résistante aux insectes à partir de la culture de cellules ou de tissus, de telle façon que l'enzyme soit exprimée à des niveaux qui produisent une quantité de limonène suffisante pour être larvicide envers les larves de Diabrotica undecimpunctata howardi ou inhiber leur capacité à s'alimenter ;
et éventuellement
(d) la reproduction sexuée ou clonale des plantes obtenues à l'étape (c) de telle manière qu'au moins une copie de la séquence codant pour la limonène synthase fournie par la cassette d'expression soit présente dans les cellules de la descendance de la reproduction et de telle manière que l'enzyme soit exprimée à des niveaux qui produisent une quantité de limonène suffisante pour être larvicide envers les larves de Diabrotica undecimpunctata howardi ou inhiber leur capacité à s'alimenter.

11. Méthode selon la revendication 10 comprenant :
(a) la culture de cellules ou de tissus de plantes ;
(b) l'introduction dans les cellules ou dans les cellules de la culture de tissus d'au moins une copie d'une cassette d'expression comprenant un gène de structure codant pour la limonène synthase, lié de manière fonctionnelle à des séquences régulatrices de plante qui entraînent l'expression de l'enzyme dans les cellules ; et
(c) la régénération de plantes entières résistantes aux insectes à partir de la culture de cellules ou de tissus ;
(d) suivie par le croisement sexué de la plante résistante aux insectes avec une plante d'un taxon sensible aux insectes ;
(e) la récupération du matériel reproducteur à partir de la descendance du croisement ; et
(f) la culture de plantes résistantes aux insectes à partir du matériel reproducteur ;
et éventuellement :
(g) la réalisation de rétrocroisements (backcross) de la descendance résistante aux insectes avec des plantes d'un taxon sensible aux insectes ; et
(h) la sélection de l'expression de la résistance aux insectes, ou d'un gène marqueur associé, ou de la production de limonène dans la descendance du rétrocroisement ;
jusqu'à ce qu'un pourcentage souhaité des caractéristiques des plantes sensibles du taxon sensible soit présent en même temps que le gène de la limonène synthase conférant la résistance aux insectes.

12. Méthode selon la revendication 10 ou 11 dans laquelle la plante est une espèce de Monocotylédones choisie parmi le maïs, le blé, le riz, l'avoine, le seigle et le sorgho.

13. Méthode selon la revendication 10 ou 11 dans laquelle la plante est une espèce de Dicotylédones choisie parmi le soja, le tournesol, le colza, la luzerne, le coton, le melon, le concombre, la laitue, le poivron et la tomate.

14. Méthode selon la revendication 10 ou 11 dans laquelle la séquence promotrice est un promoteur tissu-spécifique.

15. Méthode selon la revendication 14 dans laquelle le promoteur tissu-spécifique est un promoteur spécifique de la racine.
